(19) Europäisches Patentamt
European Patent Office
Office européen des brevets

(11) **EP 4 299 717 A1**

(12) **EUROPEAN PATENT APPLICATION**
published in accordance with Art. 153(4) EPC

(43) Date of publication:
**03.01.2024 Bulletin 2024/01**

(21) Application number: **21927415.6**

(22) Date of filing: **11.05.2021**

(51) International Patent Classification (IPC):
*C12N 1/20* (2006.01)          *C12N 1/02* (2006.01)
*C12Q 1/06* (2006.01)          *C12R 1/01* (2006.01)

(52) Cooperative Patent Classification (CPC):
Y02A 50/30

(86) International application number:
**PCT/CN2021/093000**

(87) International publication number:
**WO 2022/178982 (01.09.2022 Gazette 2022/35)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **23.02.2021 CN 202110200230**

(71) Applicant: **Gu, HaiYing
Ningbo, Zhejiang 315000 (CN)**

(72) Inventor: **Gu, HaiYing
Ningbo, Zhejiang 315000 (CN)**

(74) Representative: **López Camba, Emilia
SILEX IP
C/ Poeta Joan Maragall 9, Esc. Izq., 3o Izq.
28020 Madrid (ES)**

(54) **BACTERIAL COLONY FORMATION METHOD FOR SENSITIVE AND RAPID CULTURE OF HELICOBACTER PYLORI AND EFFECT EVALUATION METHOD FOR SAME**

(57)     Provided are a bacterial colony formation method for sensitive and rapid culture of H. pylori and an effect evaluation method for the same. The bacterial colony formation method comprises primary isolation, cryopreservation and recovery, growth of a single bacterial colony, and subculture. The effect evaluation method provides an effect evaluation index for the H. pylori culture method.

EP 4 299 717 A1

Description

TECHNICAL FIELD

The present invention relates to microbial culture technology, not limited to *Helicobacter pylori*(*H. pylori*),specifically*,* colony formation methods for *H. pylori* based on sensitive and rapid culture and evaluation methods.

BACKGROUND

*H. pylori* is a microaerophilic and the most common pathogenic bacterium that may continuously colonize human gastrointestinal mucosa and cause various gastritis, duodenitis, and peptic ulcers. It is also closely related to gastric cancer and gastric MALT lymphoma. The infection rate of H. pylori may reach over 50%. It is also related to parenteral infections, such as pneumonia, bacteremia, and serum glycated hemoglobin level. Therefore, it is necessary to diagnose *H. pylori* infection. There are many techniques for diagnosing the H. pylori infection, including screening tests and confirmation tests. The screening tests include a urease test, pathological tissue morphology examination, a UBT test, serum antibodies, stool antigens, and molecular biological techniques, etc. The confirmation tests include only H. pylori isolation culture and immunohistochemistry techniques. The H. pylori isolation culture is the most specific method of all diagnostic techniques. Conventional pathological morphology diagnosis may not exclude colonization or infection of campylobacteria and other helicobacter similar to *H. pylori.* Due to the presence of other urease positive bacteria in stomach, the UBT and urease tests may produce false positive results, the serum antibodies may reflect past infections, the stool antigens have poor sensitivity, the molecular biological techniques have high requirements, and all these techniques cannot provide reliable drug sensitivity test results. The *H. pylori* isolation culture is a "gold standard" for these diagnostic methods, and is a necessary process for phenotypic drug sensitivity tests and genotyping. In order to prevent and treat diseases caused by *H. pylori* infection, rapid isolation culture of H. pylori is of great significance. The culture includes primary isolation and subculture. Bacterial colony formation includes bacterial colony formation for primary isolation from a human body sample and bacterial colony formation for subculture. The primary isolation involves inoculating tissue samples containing living cells of H. pylori in a suitable isolation culture medium and allowing the cells to grow as many bacterial colonies as possible. The subculture involves inoculating *H. pylori* culture in a subculture medium and allowing its good growth. The isolation culture medium and the subculture medium should be different. The tissue samples usually contain miscellaneous bacteria other than H. pylori, and antibiotics that inhibit the growth of the miscellaneous bacteria need to be added to the isolation culture medium without affecting the growth of *H. pylori.* There is no need to add antibiotics to the subculture medium, and its other components may be different from that of the isolation culture medium. The subculture medium is used mainly for analysis on biological characteristics of *H. pylori,* phenotypic drug sensitivity tests, and other tests. The subculture includes the growth of bacterial colonies in a plate culture medium and bacterial colonies recovered from cryopreserved culture. The recovery rate of subculture is affected by factors such as medium components and stability. There are two important factors that affect initial isolation results of H. pylori: isolation recovery rate and isolation time. The isolation recovery rate is determined by isolation sensitivity and is affected by many factors, including whether a patient is treated using antibiotics or the like, location and quantity of collected material, material collection and sample processing method and process, transportation of culture medium, isolation culture medium, isolation culture conditions, etc., which result in significant changes in initial isolation culture sensitivity (60-90%). The isolation culture medium inhibits the growth effect of miscellaneous bacteria in gastrointestinal mucosa, which will affect the isolation recovery rate of H. pylori. Although there are many media for culturing H. pylori, rapid and sensitive isolation methods have not been reported yet. H. pylori is isolated from gastric mucosal tissue samples using a Columbia agar horse blood culture medium for 2-10 days, and H. pylori is isolated using a GC agar culture medium for 4-10 days. The essence of rapid isolation culture is to rapidly grow samples containing culturable living cells of H. pylori in a suitable culture medium to form bacterial colonies of *H. pylori.* Factors that affect the isolation culture of H. pylori are closely related to the isolation culture medium. Another important problem is that, after the initial isolation of H. pylori is successful, but the subculture fails, the drug sensitivity tests or the like will not be completed. There are literature reports that the survival rate (drug sensitivity completion rate) of subculture is only 84.8%, indicating that the subculture method for *H. pylori* has significant deficiencies. There is no literature report on the use of the initial isolation culture medium as the subculture medium for culture so as to sensitively and rapidly form bacterial colonies of H. pylori. There is no effect evaluation method for the bacterial colony formation method for sensitive and rapid isolation culture of *H. pylori.* The effect of the culture medium should meet the formula: primary culture medium=subculture medium.

## SUMMARY

### Technical problems

An object of the present invention is to provide a sensitive and rapid colony formation method for *H. pylori*, which has the same good efficiency as primary isolation and subculture.

Another object of the present invention is to provide an evaluation method for *H. pylori* culture,including multiple evaluation indexes of its effectiveness.

Solutions to technical problems

In order to achieve the objects of the present invention, in a first aspect, at first, a sensitive and rapid colony formation method for *H. pylori* provided in the present invention includes the following steps:

I, primary isolation

> step 1, opening a kit for Gu' s Medium for Rapid Isolation of *Helicobacter pylori*, (TianKuo, Xunjian Biotechnology Co., Ltd,Ningbo, China) , taking out Gu's plates , performing vacuum-sealed package, and storing the Gu's plate under waterproof and dark conditions;

> step 2, taking two zirconium beads with a particle size of 3-6 mm (Zirconium beads,Xinzhi Biotechnology Co., Ltd,Ningbo, China) , and placing them in a 1-2 mL EP tube for sterilization;

> step 3, opening a kit for Gu' s Preservation Medium for *Helicobacter pylori* (TianKuo, XunjianBiotechnology Co., Ltd,Ningbo, China) , taking a 0.5-1.0 mL Gu's transport medium , adding it into a sterile EP tube ;

> step 4, Suspected individuals with *H. pylori* infection are required to have not been treated with PPI and antibiotics for at least 20 days. Gastrointestinal tissue samples are taken and placed into the EP tube in step 3. The tissue samples are completely immersed in the Gu's transport medium, tightly capped, and stored at 2-8 °C for a storage period of no more than 10 days;

> step 5, grinding the Gu's transport medium containing the tissue samples in a tissue grinder (Tissue grinder , Xinzhi Biotechnology Co., Ltd,Ningbo, China) for 30-60 s;

> step 6, opening the vacuum-sealed package, taking out one Gu's plate, inoculating 100-200 $\mu$L ground sample on the Gu's plate for culturing of *H. pylori* under sterile conditions at a local temperature below 40 °C; culturing under microaerobic conditions (Anoxomat; MARTMicrobiology BV, Drachten, Netherlands) ,at humidity of 100% and 35-37 °C for 48-120 hours, observing the colony morphology, where in typical colony morphology is purple, circular, or light purple droplet-like spreading may appear;

II, cryopreserved specimens and recovery
step 7, Storing the liquid samples of *H. pylori* at -70-80° C, drawing 100-200 $\mu$L of the samples after thawing, and performing recovery culture according to the method in step 6;

III, formation of single colonies
step 8, picking several the *H. pylori* colonies from a fresh culture to sterile physiological saline, and adjusting turbidity visually to match a 1.0 McFarland standard with a photometric device <DensiCHEK,bioMerieux Inc., USA) , performing 1-fold serial dilution to over 13 sterile tubes; opening the vacuum-sealed package, taking out 2 Gu's plates for rapid culture of *H. pylori*, drawing a line on the center of its back, and evenly dividing it into 2 areas; taking 20$\mu$L diluted bacterial suspension (such as tube 16 ) and inoculating it separately in the agar medium area marked on the back of the plate , continuously and densely spreading with a sterile loop ; then culturing for 48 to 120 hours according to the method in step 6 and observing the formation of individual colonies on the agar medium plates;

IV, single-colony subcultures
step 9, the vacuum-sealed package, taking out one Gu's plate for rapid culture of *H. pylori*, drawing a cross line on the center of its back, and evenly dividing it into 4 areas, picking a single colony from a 72h old culture and spreading it in a continuous dense circle on an area of the plate with a diameter of 13-17mm; inoculating at a local temperature below 40 °C; culturing under microaerobic conditions with a thumidity of 100% at 35-37°C for 24 hours.Under sterile conditions, each plate can be inoculated with 4 single colonies.

In the present invention, the microaerobic conditions refer to: 3-6% $O_2$, 5-10% $CO_2$, 5-10% $H_2$ and 74-87% $N_2$.

Preferably, the temperature conditions for culture are: 37 °C, and the microaerobic conditions are: 5% $O_2$, 10% $CO_2$, 5% $H_2$, and 80% $N_2$, or 5% $O_2$, 7.6% $CO_2$, 7.6% $H_2$ and 79.8% $N_2$.

In the aforementioned method, the culture plate is vacuum -sealed packaged to meet the waterproof and dark conditions.

In the aforementioned method, a local temperature for inoculation under sterile conditions is below 40 °C.

In the aforementioned method, the transport medium contains zirconium beads.

In the aforementioned method, the samples are placed into the EP tube containing zirconium beads as soon as possible, and the EP tube is completely immersed in the transport medium, the tube is tightly capped, and then stored at 2-8°C for preservation. it at 2-8° C.

In the aforementioned method, the EP tube samples containing zirconium beads are ground for 30-60 seconds.

In the aforementioned method, formation of single colonies involves picking several the *H. pylori* colonies from a fresh culture to sterile physiological saline, and adjusting turbidity visually to match a 1.0 McFarland standard with a photometric device, performing 1-fold serial dilution to over 13 sterile tubes.

In the aforementioned method, single-colony subculture involves picking a single colony from a 72h old culture and spreading it in a continuous dense circle on an area of the plate with a diameter of 13-17mmand microaerobic culture for 24 hours.

In the present invention, the samples come from digestive systems of patients with H. pylori infection (such as gastrointestinal tissue, stool, or saliva), and the infected persons have not been treated with PPI and antibiotics for at least 20 days; gastrointestinal tissue samples are taken using endoscopic biopsy forceps.

In a second aspect, the present invention provides an evaluation method for the effect of *H. pylori* culture , where the evaluation index for the effect of *H. pylori* culture includes but is not limited to pollution inhibition rate, isolation rate, survival rate of strains recovered from tissue,recover rate of cryopreserved strains, single-colony formation sensitivity, sensitivity ratio of single colony formation in different incubation periods, the amount of cells subcultured by a single colony and amount of cells mixed with multiple single colonies.

The effect of culture medium should meet the formula: initial isolation culture medium=subculture medium.

Further, when the evaluation index is the pollution inhibition rate, the evaluation method includes:

1) inoculating Escherichia coli ATCC25922 and Staphylococcus aureus ATCC 25923 on blood agar plates, respectively, and culturing at 37°C for 16-18 h, preparing the cultured Escherichia coli ATCC25922 and Staphylococcus aureus ATCC 25923 with sterile physiological saline, respectively, and diluting to a 17th gradient, and taking 10 μL of the diluted bacterial liquid to be inoculated on the blood agar plates, culturing at 37°C for 24 h, and recording the numbers of colonies formed on the plate as CN1 and CN2 respectively;

2) inoculating Candida albicans ATCC 90028 on a Sabao weak medium plate, and culturing at 37°C for 16-18 h, preparing a bacteria solution of 1.0 McF from cultivated Candida albicans ATCC 90028 with sterile physiological saline, diluting it to a 11th gradient continuously, inoculating 10 μL of the diluted bacteria solution on the Sabao weak medium plate, and culturing at 37°C for 24 h, and recording the number of colonies formed on the plate as CN3;

3) drawing a Y-shaped line in the center of the back of a isolation plate, dividing the plate into three areas equally, inoculating 10 μL of the diluted Escherichia coli ATCC25922, Staphylococcus aureus ATCC 25923 and Candida albicans ATCC 90028 in the three areas respectively, and then culturing them for 72 h under microaerobic conditions of 3-6% $O_2$, 5-10% $CO_2$, 5-10% $H_2$ and 76-86% $N_2$, with a temperature of 35-37°C and a humidity of 100%, and recording a total number of colonies in the three areas as CN;

4) calculating the pollution inhibition rate according to the following formula:

$$\text{pollution inhibition rate (\%)}=1- \text{colony growth rate}$$

$$\text{colony growth rate (\%)}= CN/(CN1+CN2+CN3)\times100\%$$

pollution inhibition rate ≥99% indicates high culture efficiency.

Further, when the evaluation index is the strain isolation rate, the evaluation method comprises:

inoculating each sample with two different H. pylori isolation plates in parallel: a GC agar medium plate and a columbia SBA plate; the culture conditions of the two plates being the same, the culture time being 3-10 days, and carrying out identification according to a conventional method; recording the number of H. pylori positive cases obtained by using the first isolation plate as N1, recording the number of H. pylori positive cases obtained by using the second isolation plate as N2, and recording the number of H. pylori positive cases obtained by combining the two isolation plates as N3, calculating according to the following formula:

$$\text{isolation rate of the first isolation plate (\%)}=N1/N3\times100\%$$

$$\text{isolation rate of the second isolation plate (\%)}=N2/N3\times100\%$$

a plate isolation rate of 100% indicates that the isolation effect of H. pylori is good.

Further, when the evaluation index is the survival rate of strains recovered from tissue, the evaluation method comprises:

recording the number of H. pylori strains obtained by plate isolation as SN1, and recording the number of H. pylori strains obtained by subculturing each strain according to step 9 as SN2, and calculating the survival rate of strains recovered from tissue according to the following formula:

$$\text{the survival rate of strains recovered from tissue (\%)}=SN2/SN1\times100\%$$

the survival rate of strains recovered from tissue of 100% indicates that the subculture survival effect of strains is good.

Further, when the evaluation index is therecover rate of cryopreserved strains, the evaluation method comprises: operating the sample number FS1 of different strains frozen at-70°C for at least one year according to steps 7 and 8, and recording the number of successfully revived strains as FS2; and calculating thecryopreservation strain subculture revival rate according to the following formula:

$$\text{therecover \quad rate of cryopreserved strains (\%)}=FS2/FS1\times100\%$$

therecover rate of cryopreserved strains of 100% indicates that the preservation and revival effect of H. pylori is good.

Further, when the evaluation index is the single-colony formation sensitivity, the evaluation method comprises: subculturing the H. pylori model strain ATCC43504 for 72 h according to the method of step 8, and calculating the number of colonies growing in two plates, wherein the number of colonies should not be less than 20 cfu.

Further, when the evaluation index is the sensitivity ratio of single colony formation in different incubation periods, the evaluation method comprises:

1) culturing for 48 h according to the method of step 8, and recording the number of colonies formed as NC2;

2) culturing the plate for 72 h, and recording the number of colonies formed as NC3;

3) culturing the plate for 96 h, and recording the number of colonies formed as NC4;

4) if the following two conditions are met at the same time: ① $NC2/NC3\times100\%.\geq80\%$, ②$NC3/NC4\times100\%\geq90\%$, it means that H. pylori forms colonies visible to the naked eye on the culture medium plate after 48 hours, and achieves the efficiency of rapid growth, except diffuse growth colonies.

Further, when the evaluation index is the growth of a single colony subculture cell, the evaluation method comprises: culturing the H. pylori model strain ATCC43504 according to the method of step 8 for 72 h, and randomly selecting 16 single colonies to be inoculated on 4 plates for 24 h according to the method of step 9; taking 16 sterile tubes, and filling each tube with 1.3 mL sterile saline, and scraping 16 colonies with moist sterile cotton swabs and suspending in the saline of 16 sterile tubes; measuring the turbidity of each tube by a turbidimeter, wherein if the turbidity of 13 tubes is more than or equal to 1.0 McF, the plate met the requirements of rapid growth of H. pylori.

Further, when the evaluation index is the amount of cells mixed with multiple single colonies, the evaluation method comprises: culturing the H. pylori model strain ATCC43504 for 72 h according to the method of step 8, and randomly selecting 20 colonies, scraping with a wet sterile cotton swab and suspending in a sterile tube containing 1.3 mL sterile physiological saline, shaking by a shaker for 1-2 min, fully mixing, and measuring the bacterial turbidity by a turbidimeter, wherein if the bacterial turbidity is more than or equal to 1.0 McF, the plate meets the requirements of good growth of H. pylori colonies.

H. pylori was isolated, cultured and subcultured by using the Gu's rapid culture plate of H. pylori, Liofilchem sheep blood medium plate and yolk medium plate respectively. The results are shown in Table 1.

Table 1

|  | Gu's Rapid culture plate for H. pylori | Liofilchem sheep blood culture medium plate | Liofilchem yolk medium plate |
|---|---|---|---|
| Gastric biopsy specimen (n=146) |  |  |  |
| H. pylori culture positive |  |  |  |
| 2 days | 26 (37.5%) | 0 | 0 |
| 3 days | 44 (62.5%) | 43 (95.6%) | 22 (88%) |
| 5 days | 0 | 2 (4.4%) | 3 (12%) |
| Subculture survival rate |  |  |  |
| 1-2 days | 100% | - | - |
| 2-5 days | - | 51/69(73.91%) | 23/29 (79.31%) |
| Isolation rate (%) | 100% | 64.3% | 35.7% |
| Positive days | 2.0-3.0 | 3.0-5.0 | 3.0-5.0 |
| Number of colonies per plate | 3000-4000 | 500-1000 | 1000-2000 |
| Plate pollution rate | 10% | 13.3% | 12% |

Beneficial effects

Compared with that prior art, the invention has at least the following advantage: the present invention provides a colony forming method for sensitive and rapid culture of H. pylori, and the method has the same good efficiency as primary isolation and subculture. The present invention provides a powerful tool for the rapid and efficient separation and subculture of H. pylori, and at the same time provides an efficiency evaluation index for the culture method of H. pylori.

**DETAILED DESCRIPTION OF THE EMBODIMENTS**

Embodiments of the Present Invention

The following embodiments are used to illustrate the present invention, but are not intended to limit its scope. If not specifically specified, the technical means used in the embodiments are well-known conventional means for those skilled in the art, and the raw materials used are all commercially available goods.

The culture media used in the following embodiments:

A Gu's kit for rapid isolation culture, identification, drug sensitivity and preservation of H. pylori
Sabouraud's medium: BD, France.
Columbia BLOOD AGAR: OXOID, ENGLAND.

GC agar medium: BD, France.
Liofilchemsheep blood culture medium plate: Liofilchem, Italy.
Liofilchem egg yolk culture medium plate: Liofilchem, Italy.

Example 1 Bacterial colony formation method for sensitive and rapid culture of H. pylori

The method includes the following steps:

(I) primary isolation method

in step 1, a Gu's kit for rapid isolation culture, identification, drug sensitivity and preservation of H. pylori was opened, a Gu's plate for rapid culture of H. pylori was taken out and was vacuum sealed packaged, and the Gu's plate was stored under waterproof and dark conditions;
in step 2, two zirconium beads with a particle size of 3-6 mm were taken, and placed in a 1.5 mL EP tube for sterilization;
in step 3, the Gu's kit for rapid isolation culture, identification, drug sensitivity and preservation of H. pylori was opened to take a 1.0 mL Gu's culture medium for preservation of H. pylori to be placed in an EP tube with two zirconium beads;
in step 4, suspected patients with H. pylori infection were required to have not been treated with PPI and antibiotics for at least 20 days, an endoscope was used for gastrointestinal tissue sampling, and if there were abnormalities in the gastrointestinal tissue (such as ulcers, erosion, or lumps), priority should be given to sampling at the junction of normal and abnormal tissues; the samples were placed into the EP tube in step 3 as soon as possible, and were completely immersed in the culture medium, tightly capped, and stored at 2-8 °C for a storage period of no more than 10 days;
in step 5, the Gu's culture medium for preservation of H. pylori containing gastrointestinal mucosal tissue or other biological tissues was ground in a tissue grinder for 2 minutes;
in step 6, the waterproof, dark vacuum sealed package was opened to take out one Gu's plate for rapid culture of H. pylori (Gu's kit for rapid isolation culture, identification, drug sensitivity and preservation of H. pylori), 100 $\mu$L ground sample was sucked and inoculated on the Gu's plate for rapid culture of H. pylori under sterile conditions at a local temperature below 40 °C; culturing was performed under microaerobic conditions (5% $O_2$, 7. 6% $CO_2$, 7. 6% $H_2$, 79.8%$N_2$) with a humidity of 100% at 35-37 °C for 48-120 hours, the colony morphology observed, wherein typical colony morphology was purple, circular, or diffusion colony morphology may appear;
(II), cryopreservation and recovery

in step 7 (cryopreservation and recovery method), liquid samples of H. pylori were cryopreserved at -80 °C, and after thawing, 100$\mu$L samples were sucked and subjected to recovery culture according to the method in step 6;
in step 8 (single colony growth method for strain): H. pylori strains were cryopreserved according to step 7 (clinical strains according to step 6) and H. pylori strains were subcultured for 18 hours, the H. pylori colonies were mixed with sterile physiological saline to prepare a bacterial solution with a turbidity of 1.0 McF, and it was continuously diluted for multiple times to above the 16th gradient; the vacuum sealed package was opened to take out 2 plates for rapid culture of H. pylori, a line was drawn on the center of its back, and it was evenly divided into 2 areas; 20$\mu$L diluted bacterial solution (such as 16 gradients) was taken to be inoculated separately in the areas of the drawn plate culture medium, a sterile inoculation ring was used for continuous and dense circular coating; then culturing was performed according to the method in step 6 for 48 to 96 hours and the growth of individual colony on the culture medium plate was observed;
in step 9 (single colony subculture method): the waterproof, dark vacuum sealed package was opened to take out one Gu's plate for rapid culture of H. pylori (Gu's kit for rapid isolation culture, identification, drug sensitivity and preservation of H. pylori), a cross line was drawn on the center of its back, and it was evenly divided into 4 or 8 areas, a sterile inoculum ring was used to scrape a single bacterial colony for continuous and dense circular coating in one area of the plate (with a diameter of 17 mm); each plate could purify 4 strains of bacteria; sterile inoculation was performed at a local temperature below 40 °C; culturing was performed under microaerobic conditions (5% $O_2$, 7.6% $CO_2$, 7.6%$H_2$, 79.8% $N_2$) with a humidity of 100% at 35-37 °C for 24 hours, and the colony morphology was observed.

Identification:

Gram staining, oxidase, catalase and urease tests and antigen detection were carried out on the colonies in steps 6 and 9 by using the Gu's kit for rapid isolation, culture, identification, drug sensitivity and preservation of H. pylori (produced by Hainan Clinical Microbiology Testing and Research Center) and antigen detection reagents. The identification results are consistent with the biological characteristics of H. pylori. The H. pylori antigen detection reagents (latex method) were purchased from Aibo Biomedical (Hangzhou) Co., Ltd.

Example 2 Evaluation Method of H. pylori Culture Efficiency

The evaluation index of H. pylori sensitive culture efficiency adopted in this embodiment includes pollution inhibition rate, strain isolation rate, survival rate of strains recovered from tissue,recover rate of cryopreserved strains, room temperature preservation colony subculture survival rate, single-colony formation sensitivity, sensitivity ratio of single colony formation in different incubation periods, growth of a single colony subculture cell and amount of cells mixed with multiple single colonies.

1. pollution inhibition rate

1) Escherichia coli ATCC25922 and Staphylococcus aureus ATCC 25923 were inoculated on blood agar plates, respectively, and cultured at 37°C for 16-18 h, the cultured Escherichia coli ATCC25922 and Staphylococcus aureus ATCC 25923 were prepared with sterile physiological saline, respectively, and diluted to a 17th gradient, and 10 μL of the diluted bacterial liquid was taken to be inoculated on the blood agar plates, cultured at 37°C for 24 h, and the numbers of colonies formed on the plate were recorded as CN1 and CN2 respectively;

2) Candida albicans ATCC 90028 was inoculated on a Sabao weak medium plate, and cultured at 37°C for 16-18 h, bacteria solution of 1.0 McF was prepared from cultivated Candida albicans ATCC 90028 with sterile physiological saline, diluted to a 11th gradient continuously, 10 μL of the diluted bacteria solution was inoculated on the Sabao weak medium plate, and cultured at 37°C for 24 h, and the number of colonies formed on the plate was recorded as CN3;

3) a Y-shaped line was drawn in the center of the back of a isolation plate, the plate was divided into three areas equally, 10 μL of the diluted Escherichia coli ATCC25922, Staphylococcus aureus ATCC 25923 and Candida albicans ATCC 90028 were inoculated in the three areas respectively, and then cultured for 72 h under microaerobic conditions of 3-6% $O_2$, 5-10% $CO_2$, 5-10% $H_2$ and 76-86% $N_2$, with a temperature of 35-37°C and a humidity of 100%, and a total number of colonies in the three areas was recorded as CN;

4) the pollution inhibition rate was calculated according to the following formula:

$$\text{pollution inhibition rate (\%)} = 1 - \text{colony growth rate}$$

$$\text{colony growth rate (\%)} = CN/(CN1+CN2+CN3) \times 100\%$$

pollution inhibition rate ≥99% indicates high culture efficiency.

2. Isolation rate,

according to the method in Embodiment 1, each sample was inoculated with two different H. pylori isolation plates in parallel: a GC agar medium plate and a columbia SBA plate; the culture conditions of the two plates were the same, the culture time was 3-10 days, and identification was carrying out according to a conventional method; the number of H. pylori positive cases obtained by using the first isolation plate was recorded as N1, the number of H. pylori positive cases obtained by using the second isolation plate was recorded as N2, and the number of H. pylori positive cases obtained by combining the two isolation plates was recorded as N3, calculation was made according to the following formula:

$$\text{isolation rate of the first isolation plate (\%)} = N1/N3 \times 100\%$$

$$\text{isolation rate of the second isolation plate (\%)} = N2/N3 \times 100\%$$

a plate isolation rate of 100% indicates that the isolation effect of H. pylori is good.

3. survival rate of strains recovered from tissue

the number of H. pylori strains obtained by plate isolation was recorded as SN1, and the number of H. pylori strains obtained by subculturing each strain according to step 8 according to the method in Embodiment 1 was recorded as SN2, and the survival rate of strains recovered from tissue was calculated according to the following formula:

$$\text{the survival rate of strains recovered from tissue (\%)} = SN2/SN1 \times 100\%$$

the survival rate of strains recovered from tissue of 100% indicates that the subculture survival effect of strains is good.

4. recover rate of cryopreserved strains

the sample number FS1 of different strains frozen at -70°C for at least one year was operated according to the method in Embodiment 1, and the number of successfully revived strains was recorded as FS2; and therecover rate of cryopreserved strains was calculated according to the following formula:

$$\text{therecover rate of cryopreserved strains (\%)} = FS2/FS1 \times 100\%$$

therecover rate of cryopreserved strains of 100% indicates that the preservation and revival effect of H. pylori is good.

5. single-colony formation sensitivity

the H. pylori model strain ATCC43504 was subcultured for 72 h according to the method in step 8 in Embodiment 2, wherein the number of colonies on the two plates were 36 cfu.

6. sensitivity ratio of single colony formation in different incubation periods

culturing was carried out for 48 h according to the method of step 8, and the number of colonies formed was recorded as NC2; the above plate was further cultured for 72 h, and the number of colonies formed was recorded as NC3; the plate was further cultured for 96 h, and the number of colonies formed was recorded as NC4; if the following two conditions are met at the same time: ①NC2/NC3×100%.≥80%, ②NC3/NC4×100%≥90%, it means that H. pylori forms colonies visible to the naked eye on the culture medium plate after 48 hours, and achieves the efficiency of rapid growth. Gu's rapid culture plate for H. pylori NC2/NC3=87%, NC3/NC4=92%.

7. Growth of a single colony subculture cell

the H. pylori model strain ATCC43504 was cultured according to the method of step 8 for 72 h, and 16 single colonies were randomly selected to be inoculated on 4 plates for 24 h according to the method of step 9; 16 sterile tubes were taken and each tube was filled with 1.3 mL sterile saline, and 16 colonies were scraped with moist sterile cotton swabs and suspended in the saline of 16 sterile tubes; the turbidity of each tube was measured by a turbidimeter, wherein if the turbidity of 13 tubes is more than or equal to 1.0 McF, the plate met the requirements of rapid growth of H. pylori. The cell growths of a single colony subcultured on the Gu's rapid culture plate of H. pylori were 4.0, 4.0, 4.0, 4.0, 4.0, 4.0, 4.0, 3.0, 4.0, 3.2, 3.9, 2.8, 4.0, 1.3 and 3.2 respectively.

8. amount of cells mixed with multiple single colonies

the H. pylori model strain ATCC43504 was cultured for 72 h according to the method of step 8, and 20 colonies were randomly selected, scraped with a wet sterile cotton swab and suspended in a sterile tube containing 1.3 mL sterile physiological saline, the tube was shaken by a shaker for 1-2 min to be fully mixed, and the bacterial turbidity was measured by a turbidimeter; wherein if the bacterial turbidity is more than or equal to 1.0 McF, the plate met the requirements of good growth of H. pylori colonies. The growth amount of mixed colony cells in the Gu's rapid culture plate of H. pylori was 1.1.

Although the present invention has been described in detail with general descriptions and specific embodiments, it is obvious to those skilled in the art that some modifications or improvements can be made on the basis of the present invention. Therefore, these modifications or improvements made without departing from the spirit of the invention belong to the scope of the invention.

## Claims

1. A bacterial colony formation method for sensitive and rapid culture of H. pylori, comprising the following steps:

    I, primary isolation

    step 1, opening a Gu's kit for rapid isolation culture, identification, drug sensitivity and preservation of H. pylori, taking out a Gu's plate for rapid culture of H. pylori, performing vacuum sealed packaging, and storing the Gu's plate under waterproof and dark conditions;
    step 2, taking two zirconium beads with a particle size of 3-6 mm, and placing them in a 1-2 mL EP tube for sterilization;
    step 3, opening a Gu's kit for rapid isolation culture, identification, drug sensitivity and preservation of H. pylori, taking a 0.5-1.0 mL Gu's culture medium for preservation of H. pylori, and placing it in an EP tube for sterilization;
    step 4, requiring suspected patients with H. pylori infection to have not been treated with PPI and antibiotics for at least 20 days, taking gastrointestinal tissue samples and placing them into the EP tube in step 3, completely immersing the tissue samples in the culture medium, tightly capping, and storing the samples at 2-8 °C for a storage period of no more than 10 days;
    step 5, grinding the Gu's culture medium for preservation of H. pylori containing the tissue samples in a tissue grinder for 1-2 minutes;
    step 6, opening the vacuum sealed package, taking out one Gu's plate for rapid culture of H. pylori, sucking 100-200 $\mu$L ground sample and inoculating it on the Gu's plate for rapid culture of H. pylori under sterile conditions at a local temperature below 40 °C; culturing under microaerobic conditions, with a humidity of 100% at 35-37 °C for 48-120 hours, observing the colony morphology, wherein typical colony morphology is purple, circular, or diffusion colony morphology may appear;

    II, cryopreservation and recovery
    step 7, cryopreserving liquid samples of H. pylori at -70~-80 °C, and after thawing, sucking 100-200 $\mu$L samples and performing recovery culture according to the method in step 6;
    III, single colony growth
    step 8, mixing the H. pylori colonies with sterile physiological saline to prepare a bacterial solution with a turbidity of 1.0 McF, continuously diluting it multiple times to above the 13th gradient; opening the vacuum sealed package, taking out 2 Gu's plates for rapid culture of H. pylori, drawing a line on the center of its back, and evenly dividing it into 2 areas; taking 20$\mu$L diluted bacterial solution and inoculating it separately in the areas of the drawn plate culture medium, using a sterile inoculation ring for continuous and dense circular coating; then culturing for 48 to 96 hours according to the method in step 6 and observing the growth of individual colony on the culture medium plate;
    IV, single colony subculture
    step 9, opening the vacuum sealed package, taking out one Gu's plate for rapid culture of H. pylori, drawing a cross line on the center of its back, and evenly dividing it into 4 or 8 areas, using a sterile inoculum ring to scrape a single bacterial colony for continuous and dense circular coating in one area of the plate, with a diameter of 13-17mm; inoculating at a local temperature below 40 °C; culturing under microaerobic conditions with a thumidity of 100% at 35-37°C for 24 hours, and observing the colony morphology;
    wherein, the microaerobic conditions refer to: 3-6% $O_2$, 5-10% $CO_2$, 5-10% $H_2$ and 76-86% $N_2$.

2. The method according to claim 1, wherein the culture plate is vacuum sealed and packaged to meet the waterproof and light-proof conditions.

3. The method according to claim 1, wherein the temperature conditions for culture are: 37 °C, and the microaerobic conditions are: 5% $O_2$, 10% $CO_2$, 5% $H_2$, and 80% $N_2$, or 5% $O_2$, 7.6% $CO_2$, 7.6% $H_2$ and 79.8% $N_2$.

4. The method according to claim 1, wherein a local temperature for inoculation under sterile conditions is below 40 °C.

5. The method according to claim 1, wherein the preservation culture medium contains zirconium beads.

6. The method according to claim 1, wherein the samples are placed into the EP tube containing zirconium beads as soon as possible, and the EP tube is completely immersed in the culture medium, the tube is tightly capped, and then placed at 2-8°C for preservation.

7. The method according to claim 1, wherein the EP tube samples containing zirconium beads are ground for 1-2 minutes.

8. The method according to claim 1, wherein single colony growth involves mixing H. pylori colonies with physiological saline to prepare a bacterial solution with a turbidity of 1.0 McF, and continuously diluting it multiple times to above a 13th gradient.

9. The method according to claim 1, wherein single colony subculture involves using a sterile inoculum ring to scrape a single colony for continuous and dense circular coating in one area of the plate and microaerobic culture for 24 hours.

10. The method according to claim 1, wherein if there are abnormalities in the gastrointestinal tissue, sampling is performed at the junction of normal and abnormal tissues;
wherein, abnormal gastrointestinal tissues comprise inflammation, ulcer, erosion or lump.

11. An effect evaluation method for culture of H. pylori, where an effect evaluation index for culture of H. pylori comprises but is not limited to pollution inhibition rate, strain isolation rate, survival rate of strains recovered from tissue, recover rate of cryopreserved strains, room temperature preservation colony subculture survival rate, single-colony formation sensitivity, sensitivity ratio of single colony formation in different incubation periods, growth of a single colony subculture cell and amount of cells mixed with multiple single colonies.

12. The method according to claim 11, wherein when the evaluation index is the pollution inhibition rate, the evaluation method comprises:

1) inoculating Escherichia coli ATCC25922 and Staphylococcus aureus ATCC 25923 on blood agar plates, respectively, and culturing at 37°C for 16-18 h, preparing the cultured Escherichia coli ATCC25922 and Staphylococcus aureus ATCC 25923 with sterile physiological saline, respectively, and diluting to a 17th gradient, and taking 10 $\mu$L of the diluted bacterial liquid to be inoculated on the blood agar plates, culturing at 37°C for 24 h, and recording the numbers of colonies formed on the plate as CN1 and CN2 respectively;
2) inoculating Candida albicans ATCC 90028 on a Sabao weak medium plate, and culturing at 37°C for 16-18 h, preparing a bacteria solution of 1.0 McF from cultivated Candida albicans ATCC 90028 with sterile physiological saline, diluting it to a 11th gradient continuously, inoculating 10 $\mu$L of the diluted bacteria solution on the Sabao weak medium plate, and culturing at 37°C for 24 h, and recording the number of colonies formed on the plate as CN3;
3) drawing a Y-shaped line in the center of the back of a isolation plate, dividing the plate into three areas equally, inoculating 10 $\mu$L of the diluted Escherichia coli ATCC25922, Staphylococcus aureus ATCC 25923 and Candida albicans ATCC 90028 in the three areas respectively, and then culturing them for 72 h under microaerobic conditions of 3-6% $O_2$, 5-10% $CO_2$, 5-10% $H_2$ and 76-86% $N_2$, with a temperature of 35-37°C and a humidity of 100%, and recording a total number of colonies in the three areas as CN;
4) calculating the pollution inhibition rate according to the following formula:

$$\text{pollution inhibition rate (\%)} = 1 - \text{colony growth rate}$$

$$\text{colony growth rate (\%)= CN/(CN1+CN2+CN3)}\times100\%$$

pollution inhibition rate ≥99% indicates high culture efficiency.

13. The method according to claim 11, wherein when the evaluation index is the strain isolation rate, the evaluation method comprises:

inoculating each sample with two different H. pylori isolation plates in parallel: a GC agar medium plate and a columbia SBA plate; the culture conditions of the two plates being the same, the culture time being 3-10 days, and carrying out identification according to a conventional method; recording the number of H. pylori positive cases obtained by using the first isolation plate as N1, recording the number of H. pylori positive cases obtained by using the second isolation plate as N2, and recording the number of H. pylori positive cases obtained by combining the two isolation plates as N3, calculating according to the following formula:

$$\text{isolation rate of the first isolation plate (\%)=N1/N3}\times100\%$$

$$\text{isolation rate of the second isolation plate (\%)=N2/N3}\times100\%$$

a plate isolation rate of 100% indicates that the isolation effect of H. pylori is good.

14. The method according to claim 11, wherein when the evaluation index is the survival rate of strains recovered from tissue, the evaluation method comprises:

recording the number of H. pylori strains obtained by plate isolation as SN1, and recording the number of H. pylori strains obtained by subculturing each strain according to step 9 as SN2, and calculating the survival rate of strains recovered from tissue according to the following formula:

$$\text{the survival rate of strains recovered from tissue(\%)=SN2/SN1}\times100\%$$

the survival rate of strains recovered from tissue of 100% indicates that the subculture survival effect of strains is good.

15. The method according to claim 11, wherein when the evaluation index is therecover rate of cryopreserved strains, the evaluation method comprises:

operating the sample number FS1 of different strains frozen at-70°C for at least one year according to steps 7 and 8, and recording the number of successfully revived strains as FS2; and calculating therecover rate of cryopreserved strains according to the following formula:

$$\text{therecover } \text{ rate of cryopreserved strains (\%)=FS2/FS1}\times100\%$$

therecover rate of cryopreserved strains of 100% indicates that the preservation and revival effect of H. pylori is good.

16. The method according to claim 11, wherein when the evaluation index is the room temperature preservation colony subculture survival rate, the evaluation method comprises:

each plate purifying 4 strains according to the method of steps 1-9; putting M different strains of H. pylori in a clean and moist container at room temperature of 25°C for 24 h or more; inoculating each strain according to the method of step 9, and recording the subculture survival number of the strains as VS, and calculating the room temperature preservation colony subculture survival rate according to the following formula:

$$\text{the room temperature preservation colony subculture survival rate (\%)= VS/4M} \times 100\%$$

the room temperature preservation colony subculture survival rate of 100% indicates that the strains of H. pylori stored at room temperature for 24 hours or more do not die.

17. The method according to claim 11, wherein when the evaluation index is the single-colony formation sensitivity, the evaluation method comprises:
subculturing the H. pylori model strain ATCC43504 for 72 h according to the method of step 8, and calculating the number of colonies growing in two plates, wherein the number of colonies should not be less than 20 cfu.

18. The method according to claim 11, wherein when the evaluation index is the sensitivity ratio of single colony formation in different incubation periods, the evaluation method comprises:

1) culturing for 48 h according to the method of step 8, and recording the number of colonies formed as NC2;
2) culturing the plate for 72 h, and recording the number of colonies formed as NC3;
3) culturing the plate for for 96 h, and recording the number of colonies formed as NC4;
4) if the following two conditions are met at the same time: ①NC2/NC3×100%.≥80%, ②NC3/NC4×100%≥90%, it means that H. pylori forms colonies visible to the naked eye on the culture medium plate after 48 hours, and achieves the efficiency of rapid growth, except diffuse growth colonies.

19. The method according to claim 11, wherein when the evaluation index is the growth of a single colony subculture cell, the evaluation method comprises:
culturing the H. pylori model strain ATCC43504 according to the method of step 8 for 72 h, and randomly selecting 16 single colonies to be inoculated on 4 plates for 24 h according to the method of step 9; taking 16 sterile tubes, and filling each tube with 1.3 mL sterile saline, and scraping 16 colonies with moist sterile cotton swabs and suspending in the saline of 16 sterile tubes; measuring the turbidity of each tube by a turbidimeter, wherein if the turbidity of 13 tubes is more than or equal to 1.0 McF, the plate meets the requirements of rapid growth of H. pylori.

20. The method according to claim 11, wherein when the evaluation index is the amount of cells mixed with multiple single colonies, the evaluation method comprises:
culturing the H. pylori model strain ATCC43504 for 72 h according to the method of step 8, and randomly selecting 20 colonies, scraping with a wet sterile cotton swab and suspending in a sterile tube containing 1.3 mL sterile physiological saline, shaking by a shaker for 1-2 min, fully mixing, and measuring the bacterial turbidity by a turbidimeter; wherein if the bacterial turbidity is more than or equal to 1.0 McF, the plate meets the requirements of good growth of H. pylori colonies.

## INTERNATIONAL SEARCH REPORT

| International application No. |
| --- |
| **PCT/CN2021/093000** |

### A. CLASSIFICATION OF SUBJECT MATTER

C12N 1/20(2006.01)i; C12N 1/02(2006.01)i; C12Q 1/06(2006.01)i; C12R 1/01(2006.01)n

According to International Patent Classification (IPC) or to both national classification and IPC

### B. FIELDS SEARCHED

Minimum documentation searched (classification system followed by classification symbols)

C12N,C12Q,C12R

Documentation searched other than minimum documentation to the extent that such documents are included in the fields searched

Electronic data base consulted during the international search (name of data base and, where practicable, search terms used)

CNABS, DWPI, SIPOABS, CNTXT, TWTXT, WOTXT, USTXT, EPTXT, JPTXT, CNKI, 万方数据库, WANFANG DATABASE, WEB OF SCIENCE, PUBMED: 幽门螺杆菌, 分离, 培养基, 评价, 谷氏幽门螺杆菌快速分离培养、鉴定、药敏、保存试剂盒, 谷海瀛, Helicobacter pylori, Isolating, medium, Evaluat+, assess+

### C. DOCUMENTS CONSIDERED TO BE RELEVANT

| Category* | Citation of document, with indication, where appropriate, of the relevant passages | Relevant to claim No. |
| --- | --- | --- |
| X | CN 106635862 A (HANGZHOU ZHIYUAN MEDICAL LABORATORY CO., LTD.) 10 May 2017 (2017-05-10) <br> see description, paragraphs 33-90 | 11-12 |
| A | CN 111139280 A (HAINAN PROVINCIAL CLINICAL MICROBIOLOGY TESTING AND RESEARCH CENTER et al.) 12 May 2020 (2020-05-12) <br> see entire document | 11-12 |
| A | CN 109880772 A (GUIZHOU MEDICAL UNIVERSITY et al.) 14 June 2019 (2019-06-14) <br> see entire document | 11-12 |
| A | WO 9618291 A1 (YOSHITOMI PHARMACEUTICAL et al.) 20 June 1996 (1996-06-20) <br> see entire document | 11-12 |
| A | 王琼等 (WANG, Qiong et al.). "幽门螺杆菌不同运送条件及培养基分离效果的比较 (Effect Of Different Transport Conditions And Media on Helicobacter Pylori Isolation)" <br> 世界华人消化杂志 (World Chinese Journal of Digestology), <br> Vol. 24, No. 8, 31 August 2016 (2016-08-31), <br> ISSN: 1009-3079, <br> pp. 1241-1246, see entire document | 11-12 |

☐ Further documents are listed in the continuation of Box C.  ☑ See patent family annex.

| * | Special categories of cited documents: |
| --- | --- |
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
| --- | --- |
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
| --- | --- |
| **05 August 2021** | **22 October 2021** |

| Name and mailing address of the ISA/CN | Authorized officer |
| --- | --- |
| **China National Intellectual Property Administration (ISA/ CN)** <br> **No. 6, Xitucheng Road, Jimenqiao, Haidian District, Beijing 100088, China** | |
| Facsimile No. **(86-10)62019451** | Telephone No. |

Form PCT/ISA/210 (second sheet) (January 2015)

## INTERNATIONAL SEARCH REPORT

| International application No. |
| --- |
| **PCT/CN2021/093000** |

**Box No. II**      **Observations where certain claims were found unsearchable (Continuation of item 2 of first sheet)**

This international search report has not been established in respect of certain claims under Article 17(2)(a) for the following reasons:

1. ☐   Claims Nos.:
   because they relate to subject matter not required to be searched by this Authority, namely:

2. ☑   Claims Nos.: **1-10,13-20**
   because they relate to parts of the international application that do not comply with the prescribed requirements to such an extent that no meaningful international search can be carried out, specifically:

   [1]   Claims 1-10 and 14-20 relate to a sensitive and rapid culture colony formation method for helicobacter pylori and an evaluation method therefor. In the specific steps, "a helicobacter pylori rapid separation culture, identification, drug sensitivity and storage kit", "a helicobacter pylori rapid culture plate" and "a helicobacter pylori preservation medium" are necessarily used; however, neither the prior art nor the present application discloses the components and content of the described product, the structures thereof and the preparation method therefor, claim 13 defines that a separation rate calculation formula relates to N1-N3, wherein N1 and N2 can be obtained by direct counting, but the present application does not recite an algorithm for obtaining N3 by synthesis according to N1 and N2. Therefore, no meaningful search can be conducted.

3. ☐   Claims Nos.:
   because they are dependent claims and are not drafted in accordance with the second and third sentences of Rule 6.4(a).

Form PCT/ISA/210 (continuation of first sheet) (January 2015)

**INTERNATIONAL SEARCH REPORT**
Information on patent family members

International application No.

**PCT/CN2021/093000**

| Patent document cited in search report | | | Publication date (day/month/year) | Patent family member(s) | | | Publication date (day/month/year) |
|---|---|---|---|---|---|---|---|
| CN | 106635862 | A | 10 May 2017 | None | | | |
| CN | 111139280 | A | 12 May 2020 | None | | | |
| CN | 109880772 | A | 14 June 2019 | CN | 109880772 | B | 08 December 2020 |
| WO | 9618291 | A1 | 20 June 1996 | US | 5929299 | A | 27 July 1999 |
| | | | | EP | 0757888 | A4 | 26 November 1997 |
| | | | | CA | 2182805 | A1 | 20 June 1996 |
| | | | | EP | 0757888 | A1 | 12 February 1997 |
| | | | | KR | 970700994 | A | 17 March 1997 |

Form PCT/ISA/210 (patent family annex) (January 2015)